# EUROPÄISCHE PATENTSCHRIFT

(11) **EP 1 467 665 B1**
(45) Veröffentlichungstag und Bekanntmachung des Hinweises auf die Patenterteilung: **02.06.2010**
(21) Anmeldenummer: 03731709.6
(22) Anmeldetag: 23.01.2003
(51) Int. Cl.: A61B 17/66

(54) **DISTRAKTIONSVORRICHTUNG FÜR KIEFERORTHOPÄDISCHE/KIEFERCHIRURGISCHE ZWECKE AM UNTERKIEFER**
DISTRACTING DEVICE FOR ORTHODONTIC/OROSURGICAL PURPOSES ON THE LOWER JAW
DISPOSITIF DE DISTRACTION POUR ORTHODONTIE OU CHIRURGIE DENTAIRE AU NIVEAU DU MAXILLAIRE INFERIEUR

(30) Priorität: 23.01.2002 CH 110022002
(43) Veröffentlichungstag der Anmeldung: 20.10.2004
(73) Patentinhaber: Triaca, Albino, 8006 Zürich (CH); Minoretti, Roger, 8006 Zürich (CH); Merz, Beat, 8126 Zumikon (CH)
(72) Erfinder: TRIACA, Albino, CH 8006 Zürich (CH); MINORETTI, Roger, CH-8006 Zürich (CH); MERZ, Beat, CH-8126 Zumikon (CH); BAUMGARTNER, Reto, CH-4412 Nuglar (CH); BRUDERER, Thomas, CH-8215 Hallau (CH); VON MENTLEN, Roger, CH-8427 Rorbas (CH)
(74) Vertreter: Grimm, Ekkehard
(86) Internationale Anmeldenummer: PCT/EP2003/000688
(87) Internationale Veröffentlichungsnummer: WO 2003/061493

(56) Entgegenhaltungen:
- US-A- 4 433 956
- US-A- 5 775 907
- US-A- 5 829 971
- PATENT ABSTRACTS OF JAPAN vol. 1997, no. 12, 25. Dezember 1997 (1997-12-25) -& JP 09 215699 A (NAGOYA RASHI SEISAKUSHO)

## Beschreibung

Die vorliegende Erfindung betrifft eine Distraktionsvorrichtung für kieferorthopädische/kieferchirurgische Zwecke am Unterkiefer zur Distraktion eines Unterkieferknochenfrontsegments oberhalb des Kinnknochens gegen den vorderen Unterkieferrand gemäß dem Oberbegriff des Anspruchs 1.

Die in Rede stehende Distraktionsvorrichtung kommt auf den Gebieten der Kieferorthopädie und der Kiefer-Gesichtschirurgie zum Einsatz. Sie hat zum Ziel, dem Behandler eine Vorrichtung an die Hand zu geben, um Knochensegmente, z.B. im Bereich der anterioren Mandibula, zu distrahieren. Insbesondere soll eine solche Vorrichtung ermöglichen, eine Kinnpartie prominenter zu gestalten oder ein anteriores Knochensegment samt darin befindlichen Zähnen zu verschieben und gegebenenfalls auch in der Achslage zu verändern.

In der Kiefer-Gesichtschirurgie besteht oft der Bedarf, ein Segment eines Knochens gezielt graduell zu verschieben bzw. in eine andere Lage zu rotieren oder beide Bewegungen zu kombinieren, wobei die Bewegung über mehrere Tage nach der Osteotomie durchgeführt wird, im Sinne einer sogenannten Kallusdistraktion.

Eine Anwendung ist die Kinnkorrektur, bei der der vordere, untere Rand des Unterkiefers abgetrennt und in einer nach vorne verschobenen Position mit Osteosyntheseschrauben wieder befestigt wird, um damit die Prominenz des Kinnes, z.B. bei einem sogenannten fliehenden Kinn, zu vergrössern und dem Patienten zu einem ästhetischeren Gesichtsprofil zu verhelfen. Typischerweise wird diese Verschiebung in einem Schritt durchgeführt, was an den nach vorne verschobenen Trennstellen zu Diskontinuitäten im Knochenprofil und damit dem von aussen sichtbaren Weichteilprofil des Unterkiefer-Unterrandes führen kann. Ausserdem werden die darüber liegenden Weichteile in einer einschrittigen Verschiebung stark angespannt und strapaziert

In einer anderen Anwendung besteht der Wunsch, ein anteriores Alveolarkammsegment des Unterkiefers samt den darin befindlichen Zähnen abzutrennen und etwas nach vorne zu verschieben und/oder zu rotieren, z.B. um bei einem Frontzahnengstand etwas mehr Platz für die Zähne zu schaffen, oder um einem gegenüber dem Oberkiefer zu kurzen Unterkiefer etwas mehr Länge zu verleihen, damit dieser bezüglich der Okklusion besser mit dem Oberkiefer zusammenpasst Solche Verschiebungen wurden in der Vergangenheit ebenfalls in einem Schritt ausgeführt. Dieses Vorgehen hat den Nachteil, dass nur eine begrenzte Bewegung möglich ist, da genügend Überlappung an den Osteotomieoberflächen bestehen bleiben muss. Zudem werden wiederum die Weichteile stark strapaziert und es kann z.B. zu Dehiszenzen der Schleimhaut im Bereich des Trennspaltes kommen.

Die US 5,829,971 beschreibt eine Vorrichtung mit den Merkmalen der Oberbegriffs des Anspruchs 1, die für eine Distraktion, des (gesamten) Unterkiefers eingesetzt werden soll, wie anhand einer in der Zeichnung dargestellten, mit CS bezeichneten Osteotomie zu erkennen ist. Die in dieser Druckschrift beschriebene Vorrichtung ist im Bereich der Zähne gehalten und ist aus zwei Teilen aufgebaut, die gegeneinander distrahiert werden können. Eine Verankerung dieser Vorrichtung erfolgt zum einen an den Zahnreihen und zum anderen im Bereich des aufsteigenden Asts des Unterkieferknochens.

Aufgabe der vorliegenden Erfindung ist es, die vorstehend angegebenen Nachteile bisheriger Vorrichtungen, wenn sie überhaupt als Vorrichtungen bezeichnet werden können, zu beseitigen und insbesondere eine Vorrichtung zu schaffen, mit der aus medizinischer Sicht die angestrebten Korrekturen des Unterkiefers im Hinblick auf ein Frontknochensegment erreicht werden können.

Gelöst wird diese Aufgabe mit einer Distraktionsvorrichtung für kieferorthopädische/kieferchirurgische Zwecke am Unterkiefer mit den Merkmalen des Anspruchs 1.

Mit der erfindungsgemäßen Vorrichtung ist es möglich, eine Verschiebung des Frontknochensegments graduell im Sinne einer Kallusdistraktion durchzuführen. Die Distraktion wird kontinuierlich durchgeführt, indem in kurz aufeinanderfolgenden Zeitintervallen die Distraktion über die beiden Distraktionselemente vorgenommen wird. Der zweite Vorrichtungsteil dient dazu, das Frontknochensegment in der Sagittal-Ebene zu verschwenken.

Mit der erfindungsgemäßen Vorrichtung ist eine graduelle Verschiebung bzw. Verschwenkung oder auch Rotation des Frontknochensegments in die gewünschte Position möglich; gegenüber bisherigen Operationsmethoden kann eine grössere Verschiebungsdistanz ermöglicht werden, da sich durch die stetige Distraktion mittels der beiden linearen Distraktionselemente die Weichteile im Verlauf der Distraktion dem geänderten Knochenverlauf anpassen können. Außerdem ist durch die Verbindung über den Kallus keine Überlappung der Osteotomieflächen erforderlich.

In einer Ausgestaltung der Distraktionsvorrichtung weisen die jeweiligen Endabschnitte des ersten Vorrichtungsteils Befestigungselemente für eine Befestigung an den Seitenzähnen auf.

Diese Endabschnitte des ersten Vorrichtungsteils können Befestigungselemente für ein Verschrauben mit Knochenschrauben am Unterkiefer aufweisen.

In einem bevorzugten Aufbau der Distraktionsvorrichtung ist das jeweilige Distraktionselement, das den Mittelabschnitt mit dem Endabschnitt des ersten Vorrichtungsteils verbindet, aus mindestens drei Elementen aufgebaut, die miteinander verbunden sind. Hierbei ist das erste Element hülsenförmig ausgebildet und darin drehbar das zweite und das dritte Element gelagert, wobei das zweite und das dritte Element jeweils mit gegenläufigen Gewinden in dem ersten Element verschraubt sind, so dass sich unter Drehung des ersten Elements in die eine oder andere Richtung des Distraktionselements verlängert oder verkürzt.

Um die Distraktionsvorrichtung für den Arzt gut handhabbar zu gestalten, ist im Bereich des jeweiligen Distraktionselements eine Trennstelle ausgebildet. Im Bereich dieser Trennstelle kann der Endabschnitt von dem Mittelabschnitt gelöst werden. Somit ist es möglich, dass der Arzt zunächst die Endabschnitte am Kieferknochen des Patienten anbringt, um erst dann die Distraktionselemente und den Mittelabschnitt zu befestigen.

Neben einem Verschrauben der Endabschnitte an dem Kieferknochen ist es auch möglich, die Endabschnitte an Zähnen zu befestigen, beispielsweise mit spangenartigen Teilen oder Klammern. Eine weitere Möglichkeit der Befestigung der Endabschnitte ist die Verschraubung über einen Stift mit einseitigem Gewinde, wobei auf dem anderen, dem Schraubgewinde gegenüberliegenden Ende eine Hülse oder ein Röhrchen aufgesteckt wird, in das der Endabschnitt des ersten Vorrichtungsteils eingeschoben wird. Eine solche Befestigung mit einem Röhrchen oder einer Hülse kann auch dadurch erfolgen, dass ein solches Röhrchen oder eine solche Hülse an einem um einen Zahn herum gelegtes Metallband, mit der Achse des Röhrchens oder der Hülse etwa horizontal ausgerichtet, befestigt, beispielsweise angeschweisst, wird. In dieses Röhrchen oder diese Hülse kann dann wiederum der Endabschnitt des ersten Vorrichtungsteils eingeschoben und gegebenenfalls fixiert werden.

Das zweite Vorrichtungsteil kann als Scharnier ausgebildet werden mit zwei Scharnierhälften, wobei dann die eine Scharnmierhälfte dem Kinnknochen und die andere Scharnierhälfte dem zu distrahierenden Frontknochensegment zugeordnet ist.

Bevorzugt sollte die Scharnierachse etwa parallel zu der Okklusionsebene und senkrecht zu der Sagittalebene verlaufen.

Um die Handhabung eines solchen Scharniers zu erleichtern, kann dieses einen Anschlag zur Begrenzung des Schwenkbereichs der Scharnierhälften aufweisen, so dass sich das Scharnier nur in der erforderlichen Richtung, zur lagemäßigen Korrektur des Frontknochensegments, verschwenkbar ist.

Die Befestigungsteile des zweiten Vorrichtungsteils können durch Bohrungen gebildet sein. Solche Bohrungen können die Form eines Langlochs oder mehrerer Langlöcher annehmen, so dass im Bereich der Langlöcher eine variable Befestigungsstelle möglich ist, die durch den Arzt noch während der Operation den Gegebenheiten entsprechend ausgewählt werden kann.

Ein solches Langloch kann durch sich teilweise überlappende Bohrungen gebildet sein, so dass diskrete Befestigungspositionen dem Arzt vorgegeben sind.

In einer weiteren Ausgestaltung des zweiten Vorrichtungsteils ist in das Langloch eine Führungshülse eingesetzt, die entlang des Langlochs verschiebbar ist, eine solche Führungshülse weist eine Bohrung oder einen Schlitz auf, in die eine Befestigungsschraube einsetzbar ist, mit der eine Befestigung,der Führungshülse am Frontknochensegment möglich ist. Eine solche Führungshülse könnte auch so ausgestaltet sein, dass sie in dem Langloch festklemmbar ist

Für eine Verstellung oder Verschiebung der Führungshülse, und damit des daran gehaltenen Knochenteils, kann ein Seilzug daran angreifend vorgesehen sein, mittels dem der diese Führungshülse tragende Schenkel des zweiten Vorrichtungsteils um die Scharnierachse schwenkbar ist. Ein solcher Seilzug kann dann mit einem in einem Lager gehaltenen Schraubelement gespannt werden, so dass eine Zugkraft auf die Führungshülse ausgeübt wird. Über ein Spannen dieses Seilzugs kann eine Translation des Segments zur Führungshülse und zum Scharnier erzielt werden. Dann wird mittels an den Zähnen angreifenden Federn oder ähnlichen Teilen um die Achse gekippt.

In einer weiteren Ausgestaltung des zweiten Vorrichtungsteils wird, falls das Vorrichtungsteil als Scharnier ausgebildet ist, die eine Scharnierhälfte V-förmig ausgebildet, um dann an den freien Enden der beiden Schenkel jeweils die Befestigungsteile anzuordnen. Bei einem solchen V-förmigen Aufbau der einen Scharnierhälfte kann ein zusätzlicher Befestigungspunkt an der Verbindungsstelle der beiden Schenkel vorgesehen werden.

Eine weitere, nicht erfindungsgemaßë Ausführungsform der Distraktionsvorrichtung ist dann gegeben, wenn der zweite Vorrichtungsteil an dem Mittelabschnitt des ersten Vorrichtungsteils schwenkbar und/oder drehbar gehalten ist. Bei diesem Aufbau ist somit der zweite Vorrichtungsteil an dem ersten Vorrichtungsteil gehalten und befindet sich in einer festen Zuordnung dazu. Aus kieferchirurgischer Sicht ist diese Anordnung dann bevorzugt einzusetzen, wenn der gesamte anteriore Korpus (ist das zahntragende Frontsegment inkusive Kinn) nach anterior bewegt werden muss, wobei, je nach Gesichtsprofil, das Kinn weiter nach anterior gebracht werden muss, als die Inzisalkante der Unterkieferfrontzähne.

Bei der vorstehend angesprochenen weiteren Ausführungsform kann das zweite Vorrichtungsteil als Träger ausgebildet sein, wobei dann dieser Träger mit dem Mittelabschnitt des ersten Vorrichtungsteils verbunden ist und wobei an dem Träger Befestigungsteile für das Frontknochensegment ausgebildet sind.

Weiterhin kann bei dieser weiteren Ausführungsform das zweite Vorrichtungsteil ein U-Träger sein mit zwei Schenkeln und einem die beiden Schenkel verbindenden Mittelteil, wobei der Mittelteil mit dem Mittelabschnitt des ersten Vorrichtungsteils verbunden ist und wobei die freien Enden der beiden Schenkel Befestigungsteile aufweisen.

Der zweite Vorrichtungsteil kann auch ein im Wesentlichen stabförmiger Träger sein, der an seinem freien Ende die Befestigungsteile aufweist.

Für eine Distraktion des Frontknochensegments in kranio-kaudaler Richtung wird der Träger mittels eines Verstellmechanismus in seiner Länge verlängerbar oder verkürzbar ausgestaltet; ein solcher Verstellmechanismus kann einen Spindelantrieb umfassen.

Weitere Einzelheiten und Merkmale der Erfindung ergeben sich aus der nachfolgenden Beschreibung anhand der Zeichnungen.

In den Zeichnungen:
- Fig. 1a: zeigt eine Ansicht von vorne einer ersten Ausführungsform einer Distraktionsvorrichtung zur Distraktion eines Frontknochensegments mit einem ersten Vorrichtungsteil entlang des Zahnbogens verlaufend und einem zweiten Vorrichtungsteil, wobei der zweite Vorrichtungsteil als Scharnier ausgebildet ist,
- Fig. 1b: zeigt eine Seitenansicht der Anordnung der Fig. 1 aus Richtung des Sichtpfeils 1b in Fig.1a, und zwar vor Beginn der Distraktion,
- Fig. 1c: zeigt den zweiten Vorrichtungsteil der Fig. 1 in einer vergrößerten Darstellung, und zwar zum einen in einer Draufsicht und zum anderen in einer Seitenansicht,
- Fig. 1d: zeigt eine Ansicht, vergleichbar mit derjenigen der Figur 1b, allerdings während der translatorischen Phase,
- Fig. 1e: zeigt eine Ansicht entsprechend der Fig. 1b und 1d, gegen Ende einer Behandlung, nach der das Knochensegment durch Translation vorgeschoben und anschließend leicht um seine Achse gedreht ist,
- Fig. 2: zeigt eine abgewandelte Ausführungsform des oberen Schenkels des als Scharnier ausgebildeten zweiten Vorrichtungsteils mit verschiedenen, diskreten Schraubenführungspositionen,
- Fig. 3: zeigt eine weitere Variante des oberen Abschnitts des als Scharnier ausgebildeten zweiten Vorrichtungsteils Segmentes mit einer in einem Langloch geführten Führungshülse,
- Fig. 4a: zeigt eine weitere Variante des oberen Abschnitts des Scharniers des zweiten Vorrichtungsteils, das einen zusätzlichen Seilzug mit Versteilmechanismus aufweist,
- Fig. 4b: zeigt das zweite Vorrichtungsteil der Fig. 4a schematisch in einer anatomischen Anordnung, von der Seite aus gesehen,

- Fig. 5: zeigt eine weitere Ausführungsform der Distraktionsvorrichtung, bei der der erste Vorrichtungsteil und der zweite Vorrichtungsteil direkt miteinander verbunden sind, die nur zu Erläuterungszwecken dient,
- Fig. 6.: zeigt die Distraktionsvorrichtung der Fig. 5, wie sie an einem schematisch dargestellten Unterkiefer angeordnet ist,
- Fig. 7a: zeigt die Anordnung, wie sie in Fig. 6 gezeigt ist, in einer Ansicht kranial auf den Unterkiefer mit einer Distraktion nach ventral, und
- Fig. 7b: zeigt die Anordnung, wie sie in Fig. 6 gezeigt ist, in einer Ansicht kranial auf den Unterkiefer mit einer Distraktion in transversaler Richtung.

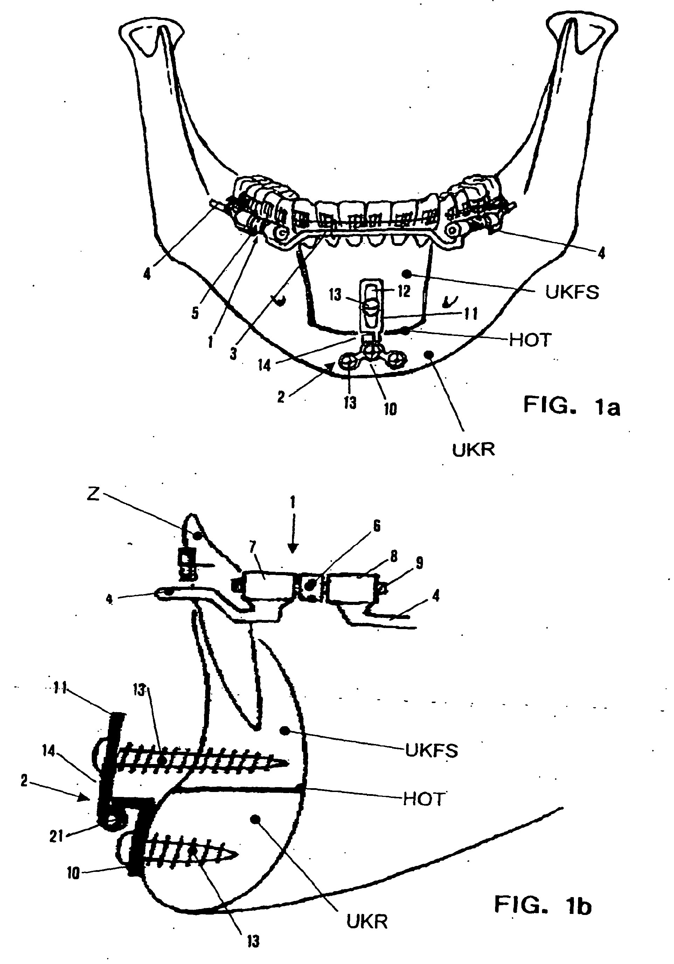

Fig. 1a zeigt eine erste Ausführungsform einer Distraktionsvorrichtung für kieferorthopädische/kieferchirurgische Zwecke am Unterkiefer zur Distraktion eines Frontknochensegments, das, wie in Fig. 1a gezeigt ist, noch die entsprechenden Frontzähne aufweist; die Vorrichtung ist aber auch dann einsetzbar, wenn die Frontzähne nicht vorhanden sind. Die Distraktionsvorrichtung umfasst einen ersten Vorrichtungsteil 1 und einen zweiten Vorrichtungsteil 2. Der erste Vorrichtungsteil 1 zeigt eine im Wesentlichen U-förmige Form, die dem Zahnbogen angenähert ist und umfasst einen Mittelabschnitt 3 sowie beidseitig daran anschließend jeweils einen Endabschnitt 4. Zwischen dem jeweiligen Endabschnitt 4 und dem Mittelabschnitt 3 ist jeweils ein lineares Distraktionselement 5 zwischengefügt. Ein solches lineares Distraktionselement ist, wie insbesondere auch die Fig. 1b verdeutlicht, aus einem ersten Element 6 und einem zweiten und dritten Element 7 und 8 aufgebaut. Das erste Element 6 ist ein hülsenförmiges Teil mit einem darin gelagerten Gewindestab 9, der sich beidseitig des ersten Elements 6 erstreckt, mit jeweils einem gegenläufigen Außengewinde. Diese beiden gegenläufigen Gewinde sind in entsprechenden Innengewinden des jeweiligen zweiten und dritten Elements 7 und 8 aufgenommen. Wie weiter in Fig. 1b zu sehen ist, sind an dem ersten Element 6 Bohrungen vorhanden, in die ein Betätigungswerkzeug zum Drehen des ersten Elements 6 und damit des Gewindestabs 9 angesetzt werden kann.

Es sollte darauf hingewiesen werden, dass das lineare Distraktionselement 5, wie es in den Figuren dargestellt ist, auch einen Aufbau, im Gegensatz zu den dargestellten Ausführungen, aufweisen kann, bei dem das mittlere, erste Element 6 eine Hülse mit zwei gegenläufigen Innengewinde-Abschnitte aufweist, in die dann das zweite und das dritte Element 7 und 8, als Gewindestab ausgebildet, eingeschraubt sind. Unter Drehung des ersten Elements 6 in die eine oder die andere Richtung kann das lineare Distraktionselement 5 verlängert oder verkürzt werden.

Die beiden Endabschnitte 4 des ersten Vorrichtungsteils 1 sind an einem Zahn oder an mehreren geeigneten Zähnen befestigt, indem um einen solchen Zahn ein Halteband herumgelegt ist, mit dem dann der Endabschnitt 4 verbunden wird.

Der Mittelabschnitt 3 ist, wie in den Fig. 1a und 1b zu sehen ist, ebenfalls an den Frontzähnen abgestützt.

Der zweite Vorrichtungsteil 2 ist in der Ausführungsform, die in den Fig. 1a und 1b dargestellt ist, als Scharnier 14 ausgebildet, mit einer ersten, unteren Scharnierhälfte 10 und einer zweiten, oberen Scharnierhälfte 11. Die untere Scharnierhälfte 10 weist darüber hinaus zwei Schenkel auf mit jeweils einer Schraubenaufnahme an den Enden und einer Schraubenaufnahme an der Verbindungsstelle der beiden Schenkel. Die zweite, obere Scharnierhälfte 11 ist als einzelner Schenkel ausgeführt, der für eine Aufnahme einer Befestigungsschraube ein Langloch 12 besitzt.

Der untere Scharnierteil 10 wird mittels üblicher Osteosyntheseschrauben, in den Figuren allgemein mit dem Bezugszeichen 13 bezeichnet, am Kieferknochen verschraubt. Der obere Schenkel 11 wird dagegen, wie Fig. 1a verdeutlicht, im Bereich eines losgetrennten Unterkieferknochensegments UKFS verschraubt.

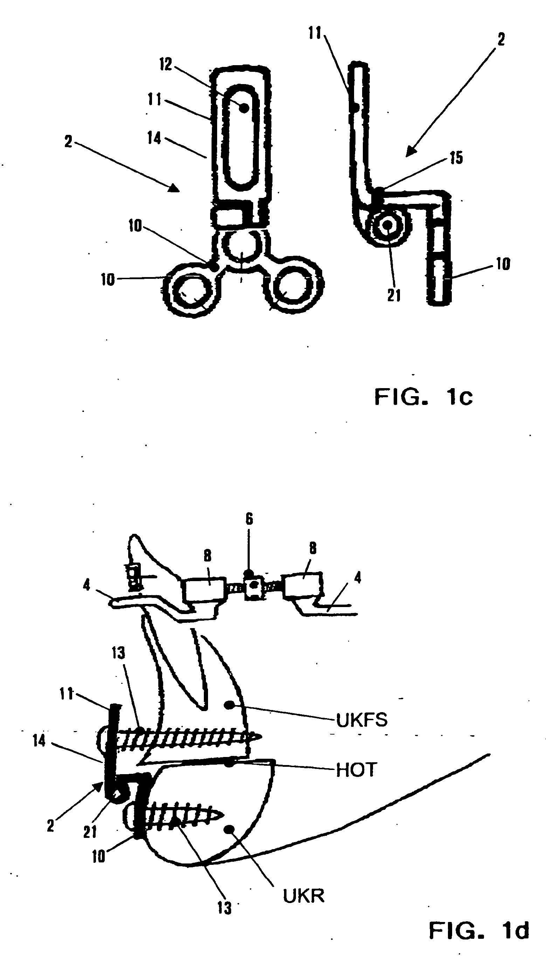

Es sollte auch darauf hingewiesen werden, dass der zweite Vorrichtungsteil in Form des Scharniers 14 einen Anschlag 15 aufweist, der in der seitlichen Ansicht des Scharniers 14 in der Fig. 1c zu sehen ist. Dieser Anschlag wird durch eine Stirnkante der unteren Scharnierteilhälfte 10 gebildet, gegen die die zweite, obere Scharnierhälfte 11 anstößt, so dass ein Verschwenken der beiden Scharnierhälften 10, 11, aus Richtung des oberen Scharnierteils 11 gesehen in Uhrzeigerrichtung, begrenzt wird. Ein solcher Anschlag ist für den Arzt bzw. Chirurgen hilfreich, um die Grundstellung, wie sie in Fig. 1b dargestellt ist, beizubehalten.

Durch das Langloch 12 in der oberen Scharnierhälfte 11 kann der zweite Vorrichtungsteil in einem gewissen Bereich verschoben und damit den Gegebenheiten angepasst werden, so dass sie in einem gewissen Höhenbereich als Widerlager für eine Osteosyntheseschraube dienen kann. Wie aus der Seitenansicht der Fig. 1b ersichtlich ist, ist die obere Scharnierhälfte 11 in ihrer Längsachse zu der unteren Scharnierhälfte 10 verschoben, damit genügend Platz für eine Vorwärtsbewegung eines Knochensegments gegeben ist; dieser Verschiebeweg ist in Fig. 1b durch den freiliegenden Gewindeabschnitt der Schraube 13 gegeben. Dieser Aufbau wird auch anhand der vergrößerten Darstellung der Fig. 1c verdeutlicht.

Während der eine Schenkel 11 des Scharniers 14 an dem Unterkieferfrontsegment UKFS, das entlang einer Osteotomie HOT getrennt ist, verschraubt ist, ist die andere, untere Scharnierhälfte 10 an dem vorderen Unterkieferrand UKR verschraubt. Es ist darauf hinzuweisen, dass neben der Schraube 13, die in der oberen Scharnierhälfte 11 gehalten ist, und das Unterkieferfrontsegment UKFS hält, das Unterkieferfrontsegment mit dem restlichen Unterkiefer auch über die in den Zeichnungen nicht näher dargestellten Weichteilen verbunden verbleibt, sowie über den ersten Vorrichtungsteil 1, der sich, wie bereits vorstehend erwähnt, an den Zähnen abstützt.

Aufgrund dieses Aufbaus der Distraktionsvorrichtung ist die Möglichkeit einer linearen Distraktion mittels der beiden Distraktionselemente 5, die dem ersten Vorrichtungsteil 1 zugeordnet sind, gegeben, neben einer Verschwenkung des Unterkieferfrontsegments UKFS gegen den Unterkieferrand UKR, mittels des zweiten Vorrichtungsteils 2, als Scharnier 14 ausgebildet, geführt. Obwohl die zwei Distraktionselemente 5, die dem ersten Vorrichtungsteil 1 zugeordnet sind, nicht parallel ausgerichtet sind, entstehen bei der Bewegung des Mittelabschnitts 3 bei Verlängerung der Distraktionselemente 5 keine Probleme aufgrund der Elastizität der Drahtsysteme, die mittels der kieferorthopädischen Brackets an den Zähnen gehalten sind. Die Distraktionsvorrichtung ist in den Fig. 1a und 1b in einer Ausgangsstellung, vor dem Beginn der aktiven Distraktion, gezeigt.

Es ist darauf hinzuweisen, dass in den einzelnen Figuren, soweit sie dieselben oder ähnliche Ausführungsformen zeigen, für vergleichbare Bauteile jeweils dieselben Bezugszeichen verwendet sind. Auch sind in den einzelnen Figuren jeweils die Abkürzungen UKR (Unterkieferrand), HOT (Osteotomie) und UKFS (Unterkieferfrontsegment) verwendet; die Bezeichnung SZK steht für Schneidezahnkante.

In Fig. 1d erfolgt die graduelle, translatorische Vorbewegung des Frontsegementes. Diese Bewegung wird hauptsächlich durch, Drehen der oberen Scharnhierhälfte 11 zugeordneten Osteosyntheseschraube 13 erzielt, die sich unter Drehung in Einschraubrichtung in das Segment (UKFS) einbohrt und es damit nach vorne zieht. Die Vorwärtsbewegung startet ca. 5 - 7 Tage nach der Osteotomie (HOT), wobei täglich 0,5 - 1 mm nach vorne bewegt wird. Gleichzeitig wird der kieferorthopädische erste Vorrichtungsteil 1 mittels der beiden Distraktionselemente 5 so betätigt, dass eine Translation entlang der horizontalen Osteotomie (HOT) erfolgt. Somit wird, wie anhand der Fig. 1d nochmals deutlich wird, mit den Distraktionselementen 5, aber auch mit der dem oberen Scharnierteil 11 zugeordneten Osteotomieschraube 13, indem diese in den Knochen eingedreht wird, distrahiert. Das Anziehen der Osteotomieschraube 13 bewirkt, dass die Basis des Segments nach vorne gelangt. Gleichzeitig bewirken die Distraktionselemente eine Kippung des Segments um die Scharnierachse, die mit 21 bezeichnet ist.

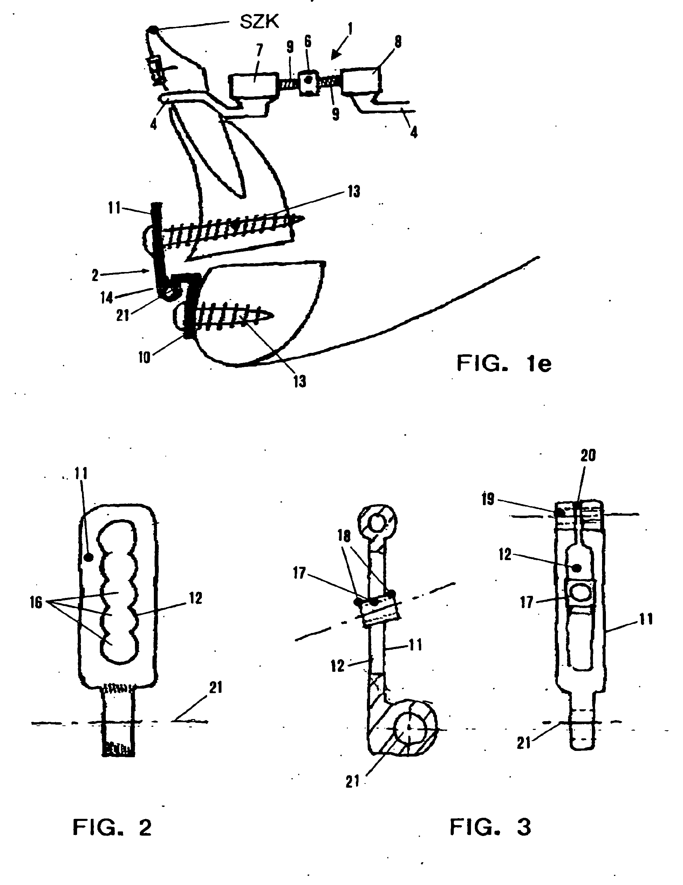

Wie die Fig. 1e zeigt, wird anschliessend an die Translation (siehe Fig. 1d) noch eine leichte Kippung durchgeführt, und zwar unter Betätigung der Distraktionselemente 5, was bezüglich der Schneidezahnkante (SZK) zu einer weiteren Vorverschiebung führt und vor allem auch die Zahnachse verändert. Dies kann z.B. dann nötig sein, wenn die Zahnachse zu sehr gegen posterior orientiert war. Die Kippbewegung wird dabei ausschliesslich über den ersten Vorrichtungsteil 1 mittels der Distraktionselemente 5 kontrolliert, während die obere Osteotomieschraube 13, die der oberen Scharnierhälfte 11 zugeordnet ist, in der Regel nicht mehr betätigt wird.

Fig. 2 zeigt eine Ausführungsform der zweiten, oberen Scharnierhälfte 11 des zweiten Vorrichtungsteils der Distraktionsvorrichtung. Bei dieser Ausführungsform ist das Langloch der oberen Scharnierhälfte 11, wie es in Fig. 1a und 1c zu sehen ist, durch mehrere überlappende Löcher 16 ersetzt. Diese überlappenden Löcher bilden mehrere diskrete Positionen, an denen die Osteotomieschraube 13 eingesetzt werden kann. Bei einer minimalen Dicke der Platte, die die Scharnierhälfte 11 bildet, ist es auch möglich, den überlappenden Bohrungen verschiedene Achsrichtungen zu geben, um der Translation eine zusätzliche, leichte Vertikalkomponente zu verleihen.

Fig. 3 zeigt eine weitere Ausführungsform der oberen Scharnierhälfte 11 des Scharniers 14, und zwar in einer geschnittenen Seitenansicht sowie in einer Draufsicht. In dieser Ausführung erfolgt die Führung der oberen Schraube durch eine Führungshülse 17, die sich entlang eines oben offenen Langloches, wiederum mit dem Bezugszeichen 12 bezeichnet, verschieben kann und in einem gewissen Winkelbereich festklemmen lässt. Die Führungshülse 17 weist an beiden Enden eine leichte Verdickung 18 auf, die ein Herausfallen der Führungshülse 17 aus dem Langloch 12 verhindert. Die Klemmung der Führungshülse 17 in der ausgewählten Position erfolgt durch eine Klemm-Schraubverbindung 19, die einen Schlitz 20 am oberen Ende des Langlochs 12 überbrückt und zusammenzieht. Die Schwenkachse des Scharniers ist in Fig. 3 mit dem Bezugszeichen 21 bezeichnet.

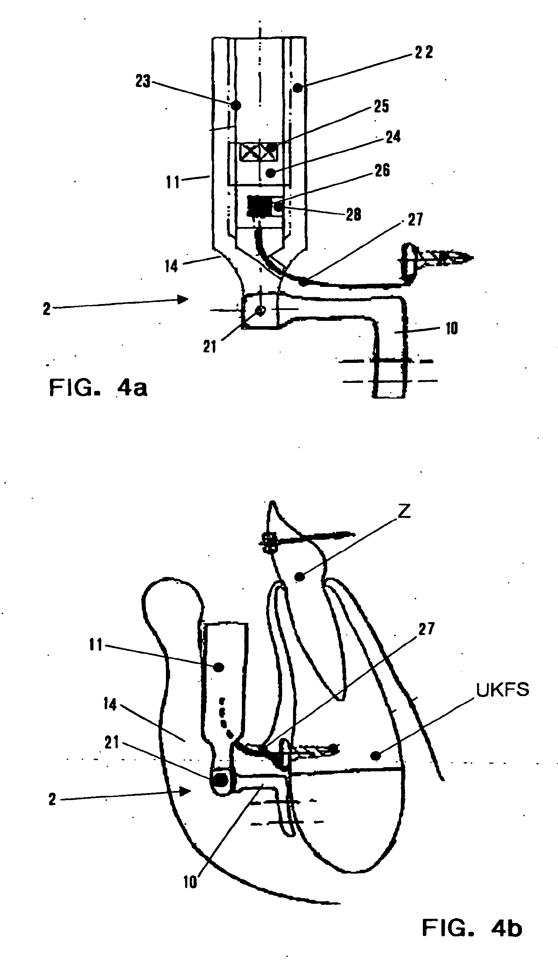

Fig. 4a zeigt eine weitere Ausführungsform des zweiten Vorrichtungsteils 2. Bei dieser Ausführungsform wird die Vorverlagerung des Knochensegmentes nicht über eine Schraube, indem diese in den Knochen eingeschraubt wird, erzielt, wie dies anhand der Fig. 1a bis 1e erläutert ist, sondern durch einen Kabelzugmechanismus. Die obere Scharnierhälfte 11 wird in dieser Ausführungsform durch einen Hohlzylinder 22 mit Innengewinde 23 gebildet. In das Innengewinde 23 ist ein kurzer Gewindebolzen 24 eingeschraubt, der von oben über die Öffnung des Hohlzylinders 22 an einem Innensechskant 25 mittels eines nicht dargestellten Inbusschlüssels gedreht werden kann. Im unteren Teil des Gewindebolzens 24 wird ein verdicktes Ende 26 eines Kabelzugs 27 gehalten; dieses verdickte Ende wird über einen seitlichen Schlitz 28 in den Gewindebolzen 24 eingesetzt. Somit wird durch das verdickte Ende 26 des Kabelzugs 27 die Drehung des Gewindebolzens 24 nicht beeinträchtigt. Der Kabelzug 27 wird im unteren Bereich umgelenkt und nach aussen geführt und ist an seinem freien Ende mit einer Osteosyntheseschraube 13 verbunden.

Fig. 4b zeigt nun diesen zweiten Vorrichtungsteil 2, wie er in Fig. 4a gezeigt ist, in einer anatomischen Anordnung. Der umgelenkte Kabelzug 27 wird über die Osteosyntheseschraube 13 am zu verschiebenden Knochensegment UKFS verschraubt und kann dieses bei entsprechender Aktivierung nach vorne bewegen. Ist das Frontsegment UKFS bis an die obere Scharnierhälfte 11 vorbewegt, kann es anschliessend analog zu der in den Fig. 1a-1e beschriebenen Ausführungsform an den Zähnen Z geführt um die Scharnierachse 21 rotiert werden. In Fig. 4b ist der erste Vorrichtungsteil der Distraktionsvorrichtung nicht näher dargestellt, es ist lediglich an dem Zahn Z ein einzelnes Bracket zu sehen.

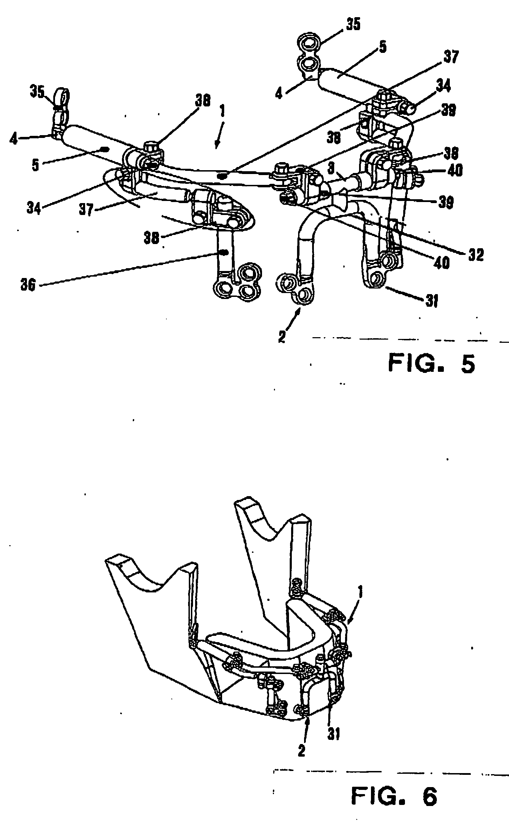

Fig. 5 zeigt einen konstruktiven Aufbau einer weiteren Distraktionsvorrichtung die kein Beispiel der Erfindung darstellt.

Bei dieser Ausführungsform der Fig. 5, die in Fig. 6 an einem schematisch dargestellten Unterkiefer angeordnet ist, ist wiederum ein erster Vorrichtungsteil 1 und ein zweiter Vorrichtungsteil 2 vorgesehen. Der erste Vorrichtungsteil 1 zeigt den typischen, U-förmigen Aufbau, der den Unterkiefer umspannt. Dagegen ist, im Gegensatz zu den Ausführungformen, wie sie anhand der vorstehend beschriebenen Figuren erläutert sind, der zweite Vorrichtungsteil 2 in Form eines U-förmigen Trägers 31 ausgeführt, mit zwei freien Schenkeln und einem Querteil 32. Dieser U-Träger 31 ist mit seinem Querteil 32 über einen Verbindungssteg 33 an dem Mittelabschnitt 3 des ersten Vorrichtungsteils 1 verbunden.

In Fig. 5 ist das jeweilige Distraktionselement zwischen dem Endabschnitt und dem Mittelabschnitt mit dem Bezugszeichen 5 bezeichnet Die Distraktion erfolgt über eine Gewindespindel an dem Schraubkopf 34. Weiterhin ist bei diesem Vorrichtungsteil eine Ramus-Befestigung 35 mit dem freien Ende des Endabschnitts 4 vorgesehen. Diese Ramus-Befestigung 35 ist so gestaltet, dass sie während der Operation gebogen und damit justiert werden kann, damit das Distraktionselement parallel zur Sagittalebene ausgerichtet werden kann. Weiterhin ist eine laterale Verankerung 36 vorgesehen, die zur Abstützung dieses ersten Vorrichtungsteils dient. Hierzu ist parallel zu einem Zwischenteil 37, zwischen dem Distraktionselement 5 und dem Mittelabschnitt 3, ein weiteres Zwischenteil 37 zwischengefügt, und zwar über jeweilige, lösbare Klemmteile oder Briden 38, das über eine solche Bride 38 gehalten ist Dieses laterale Verankerungstell 36 weist einen Befestigungsbohrungen zeigenden Endabschnitt auf. Das Zwischenteil 37 mit den beiden Klemmstellen oder Briden 38 an seinen beiden Enden ist dadurch, dass es über die Teile 38 lösbar und verschiebbar ist, flexibel einstellbar, da die Geometrien der Unterkiefer sehr unterschiedlich sein können.

Wie bereits erwähnt, wird der U-Träger 31, der den zweiten Vorrichtungsteil 2 bildet, am herausgeschnittenen Knochensegment des Unterkiefers mit seinen freien Enden befestigt, wozu an jedem Ende des U-Trägers jeweils zwei Befestigungslöcher vorhanden sind.

Damit sich bei einer Distraktion nach ventral keine Ecken oder Abstufungen im Knochen des Unterkiefers bilden, kann mit dieser Anordnung eine natürliche Form der Mandibula hergestellt werden; der Unterkiefer kann vorne gespreizt werden. Hierzu sind weitere Distraktionselemente 39 zwischen dem Mittelabschnitt 3 und dem Zwischenteil 37 eingesetzt; diese linearen Distraktionselemente können über den Betätigungskopf 40 verstellte werden. Weiterhin kann durch die Briden, mit denen die beiden weiteren Distraktionselemente 39 an dem Zwischenteil 37 und dem Mittelabschnitt 3 gehalten sind, in der Verbindung zum zweiten Vorrichtungsteil 2, d.h. dem U-Träger 31, eingestellt werden. Der U-Träger 31 selbst ist um die Sagittalachse einstellbar.

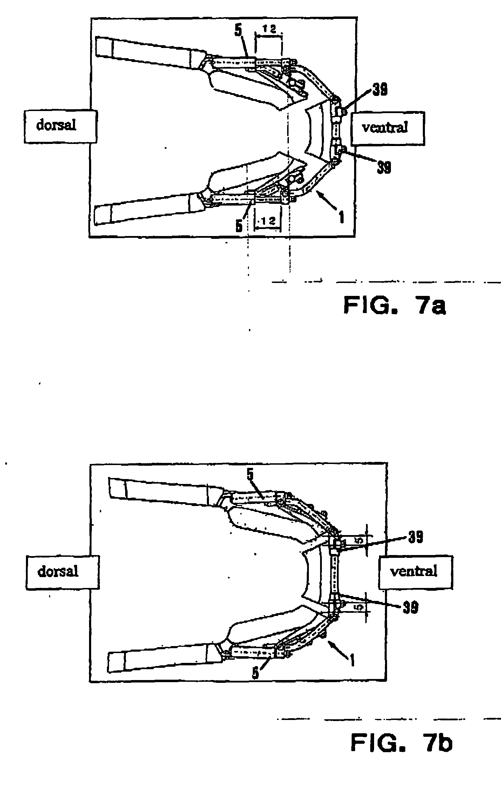

Die Fig. 7a und 7b zeigen zum einen eine Distraktion ventral und zum anderen eine Distraktion transversal.

Bei der Distraktion ventral mit der Vorrichtung, wie sie in den Fig. 5 und 6 gezeigt ist, wird durch Betätigung der beiden Distraktionselemente 5 das Knochensegment nach ventral distrahiert. In Fig. 7a ist ein Distraktionsweg von maximal 12 mm dargestellt; der erreichbare Distraktionsweg ist von der Größe des Distraktionselements 5 abhängig.

Wie anhand der Fig. 7b zu erkennen ist, kann die Form der Mandibula mit den beiden vorderen, weiteren Distraktionselementen 39 nach transversal angepasst werden. Wie in der Figur angedeutet ist, sollte eine Translation von 5 mm je weiterem Distraktionselement 39 erreichbar sein. Bei diesem Distraktionsvorgang transversal sollte berücksichtigt werden, dass sich der Unterkiefer auch im Gelenkbereich nach lateral verschiebt. Dies hat folglich Auswirkungen auf die beiden Kiefergelenke, die als Folge funktionsunfähig werden könnten. Um dies zu verhindern, müssen die Schrauben an den Trennstellen bzw. Briden 38 gelöst werden, damit sich der Winkel in diesen Gelenkverbindungen ändern kann. Nach Translation müssen die Schrauben der Briden zur Stabilisierung wieder angezogen werden.

## Patentansprüche

1. Distraktionsvorrichtung für kieferorthopädische/kieferchirurgische Zwecke am Unterkiefer zur Distraktion eines Unterkieferknochenfrontsegments (UKFS) oberhalb des Kinnknochens gegen den vorderen Unterkieferrand (UKR) mit mehreren Vorrichtungsteilen (1, 2), wobei ein erster Vorrichtungsteil (1) im Wesentlichen U-förmig, einem Zahnbogen angenähert, aufgebaut ist und einen Mittelabschnitt (3) und beidseitig davon einen Endabschnitt (4) aufweist und der jeweilige Endabschnitt (4) über ein lineares Distraktionselement (5) mit dem Mittelabschnitt (4) verbunden ist und wobei jeder Endabschnitt (4) Befestigungsmittel für eine Befestigung an den Seitenzähnen oder an Unterkiefer aufweist, **dadurch gekennzeichnet, dass** ein zweiter Vorrichtungsteil (2) vorgesehen ist, der an einem zu verschiebenden Unterkieferfrontknochensegment (UKFS) oberhalb des Kinnknochens, zu Fixierungszwecken von diesem, befestigbar ist und Befestigungsteile (12, 13) für eine Befestigung am Unterkieferrand (UKR) aufweist.

2. Distraktionsvorrichtung nach Anspruch 1, **dadurch gekennzeichnet, dass** die jeweiligen Endabschnitte (4) des ersten Vorrichtungsteils (1) Befestigungselemente für eine Befestigung an den Seitenzähnen aufweist.

3. Distraktionsvorrichtung nach Anspruch 1, **dadurch gekennzeichnet, dass** die jeweiligen Endabschnitte (4) des ersten Vorrichtungsteils (1) Befestigungselemente für ein Verschrauben mit Knochenschrauben am Unterkiefer aufweist.

4. Distraktionsvorrichtung nach Anspruch 1, **dadurch gekennzeichnet, dass** das jeweilige Distraktionselement (5) aus mindestens drei Elementen (6, 7, 8) aufgebaut ist, die miteinander verbunden sind, wobei das erste Element (6) hülsenförmig ausgebildet und darin drehbar gelagert das zweite und das dritte Element (7, 8), mit jeweils gegenläufigen Gewinden verschraubt, aufnimmt, so dass sich unter Drehung des ersten Elements (6) in die eine oder andere Richtung das Distraktionselement (5) verlängert oder verkürzt.

5. Distraktionsvorrichtung nach Anspruch 4, **dadurch gekennzeichnet, dass** im Bereich des jeweiligen Distraktionselements (5) eine Trennstelle (38) ausgebildet ist, im Bereich derer der Endabschnitt (4) von dem Mittelabschnitt (3) lösbar ist.

6. Distraktionsvorrichtung nach Anspruch 1, **dadurch gekennzeichnet, dass** das zweite Vorrichtungsteil (2) als Scharnier (14) ausgebildet ist mit zwei Scharnierhälften (10, 11), wobei die eine Scharnierhälfte (10) dem Kinnknochen zuordenbar ist und die andere Scharnierhälfte (1) dem zu distrahierenden Frontknochensegment zugeordnet ist.

7. Distraktionsvorrichtung nach Anspruch 6, **dadurch gekennzeichnet, dass** die Scharnierachse (21) etwa parallel zu der Okklusionsebene und senkrecht zu der Sagittalebene verläuft.

8. Distraktionsvorrichtung nach Anspruch 6, **dadurch gekennzeichnet, dass** das Scharnier (14) einen Anschlag (16) zur Begrenzung des Schwenkbereichs der Scharnierhälften (10, 11) aufweist.

9. Distraktionsvorrichtung nach Anspruch 6, **dadurch gekennzeichnet, dass** die Befestigungsteile durch Bohrungen gebildet sind.

10. Distraktionsvorrichtung nach Anspruch 9, **dadurch gekennzeichnet, dass** die Bohrung durch ein Langloch (12) gebildet ist.

11. Distraktionsvorrichtung nach Anspruch 10, **dadurch gekennzeichnet, dass** das Langlochs (12) durch sich teilweise überlappende Bohrungen (16) gebildet ist, so dass diskrete Befestigungspositionen gebildet sind.

12. Distraktionsvorrichtung nach Anspruch 10, **dadurch gekennzeichnet, dass** in das Langloch (12) eine Führungshülse (17) eingesetzt ist die entlang des Langlochs (12) verschiebbar ist, wobei in die Führungshülse (17) eine Befestigungsschraube einsetzbar ist.

13. Distraktionsvorrichtung nach Anspruch 12, **dadurch gekennzeichnet, dass** die Führungshülse (17) in dem Langloch (12) festklemmbar (19, 20) ist.

14. Distraktionsvorrichtung nach Anspruch 12, **dadurch gekennzeichnet, dass** an der Führungshülse (17) ein Seilzug angreift? mittels dem der diese Führungshülse (17) tragende Schenkel (11) des zweiten Vorrichtungsteils (2) um die Scharnierachse (21) schwenkbar ist.

15. Distraktionvorrichtung nach Anspruch 14, **dadurch gekennzeichnet, dass** der Seilzug mit einem in einem Lager gehaltenen Schraubelement (24) spannbar ist.

16. Distraktionsvorrichtung nach Anspruch 6, **dadurch gekennzeichnet, dass** die eine Scharnierhälfte (90) V-förmig ausgebildet ist, wobei an den freien Enden der beiden Schenkel jeweils die Befestigungsteite vorgesehen sind.

17. Distraktionsvorrichtung nach Anspruch 14, **dadurch gekennzeichnet, dass** ein zusätzlicher Befestigungspunkt an der Verbindungsstelle der beiden Schenkel vorgesehen ist.

## Claims

1. A distracting device for orthodontic/orosurgical purposes on the mandible for the distraction of a frontal bone segment of the mandible (UKFS) above the chin bone against the anterior mandibular rim (UKR) with a plurality of device parts (1, 2), wherein a first device part (1) is essentially U-shaped, approximated to a dental arch, and has a mid-segment (3) and on both sides of it an end-segment (4), and the corresponding end-segment (4) is connected to the mid-segment (4) by means of a linear distraction element (5), and wherein each end-segment (4) comprises fastening means for fastening to the lateral teeth or to the mandible, **characterized in that** a second device part (2) is provided which is adapted to be fastened to a frontal bone segment of the mandible (UFKS) to be displaced, above the chin bone, for fixing purposes thereof and which comprises fastening parts (12, 13) for fastening to the manidublar rim (UKR).

2. The distracting device according to claim 1, **characterized in that** the corresponding end-segments (4) of the first device part (1) have fixation elements for fixation to the lateral teeth.

3. The distracting device according to claim 1, **characterized in that** the corresponding end-segments (4) of the first device part (1) comprise fixation elements for a screw fixation by means of bone screws on the mandible.

4. The distracting device according to claim 1, **characterized in that** the corresponding distraction element (5) is constructed from at least three elements (6, 7, 8) which are connected to each other, wherein the first element (6) takes the form of a sleeve and accommodates, pivotably supported therein, the second and third element (7, 8), screwed with correspondingly opposite threads, such that the distraction element (5) is lengthened or shortened under rotation of the first element (6) into the one or the other direction.

5. The distracting device according to claim 4, **characterized in that** in the area of the corresponding distraction element (5) a separation point (38) is formed, in the area of which the end-segment (4) can be detached from the mid-segment (3).

6. The distracting device according to claim 1, **characterized in that** the second device part (2) takes the shape of a hinge (14) with two hinge-halves (10, 11), wherein the one hinge-half (10) can be assigned to the chin bone and the other hinge-half (11) to the frontal bone segment to be distracted.

7. The distracting device according to claim 6, **characterized in that** the hinge axis (21) is about parallel to the occlusal plane and vertical to the sagittal plane.

8. The distracting device according to claim 6, **characterized in that** the hinge (14) has a stop (15) for limiting the pivot range of the hinge-halves (10, 11).

9. The distracting device according to claim 6, **characterized in that** the fastening parts are formed by drill holes.

10. The distracting device according to claim 9, **characterized in that** the drill hole is formed by a slotted hole (12).

11. The distracting device according to claim 10, **characterized in that** the slotted hole (12) is formed by partially overlapping drill holes (16) such that discrete fastening positions are provided.

12. The distracting device according to claim 10, **characterized in that** the slotted hole (12) has inserted thereinto a guiding sleeve (17) which is movable along the slotted hole (12), a fixation screw being insertable into the guiding sleeve (17).

13. The distracting device according to claim 12, **characterized in that** the guiding sleeve (17) can be fixedly clamped (19, 20) in the slotted hole (12).

14. The distracting device according to claim 12, **characterized in that** the guiding sleeve (17) has acting thereon a cable linkage by means of which linkage the leg (11) bearing said guiding sleeve (17) and pertaining to the second device part (2) can be pivoted around the hinge axis (21) .

15. The distracting device according to claim 14, **characterized in that** the cable linkage can be tensioned by means of a screw element (24) held in a bearing.

16. The distracting device according to claim 6, **characterized in that** the one hinge-half (10) is V-shaped, wherein fastening parts are respectively provided on the free ends of the two legs.

17. The distracting device according to claim 14, **characterized in that** an additional fastening point is provided at the connection point between the two legs.

## Revendications

1. Dispositif de distraction pour orthodontie ou chirurgie dentaire au niveau du maxillaire inferieur pour la distraction d'un segment frontal de maxillaire inférieur (UKFS) au-dessus de l'os du menton contre le bord avant maxillaire inférieur (UKR) avec plusieurs parties de dispositifs (1, 2), une première pièce de dispositif (1) étant sensiblement réalisée en forme de U approximativement la forme d'une arcade dentaire et présente une section médiante (3) et des deux côté une section d'extrémité (4) et la section d'extrémité respective (4) est reliée par un élément de distraction linéaire (5) à la section médiane (4) et chaque section d'extrémité (4) présentant des moyens de fixation pour une fixation sur les dents latérales ou sur le maxillaire inférieur, **caractérisé en ce qu'**une seconde partie de dispositif (2) est prévue qui est fixée sur un segment frontal de maxillaire inférieur (UKFS) au-dessus de l'os du mention dans un but de sa fixation et présente des pièces de fixation (12, 13) pour une fixation sur le bord du maxillaire inférieur (UKR).

2. Dispositif de distraction selon la revendication 1, **caractérisé en ce que** les sections d'extrémité respectives (4) de la première partie de dispositif (1) présentent des éléments de fixation pour une fixation sur les dents latérales.

3. Dispositif de distraction selon la revendication 1, **caractérisé en ce que** les sections d'extrémité respectives (4) de la première partie de dispositif (1) présentent des éléments de fixation pour des vis pour ostéosynthèse sur le maxillaire inférieur.

4. Dispositif de distraction selon la revendication 1, **caractérisé en ce que** l'élément de distraction respectif (5) se compose d'a moins trois éléments (6, 7, 8) qui sont reliés entre eux, le premier élément (6) étant réalisé en forme de gaine et recevant le second et troisième élément (7, 8) logés de manière rotative avec des filets opposés, de sorte que sous la rotation du premier élément (6) dans une direction ou l'autre, l'élément de distraction (5) est raccourci ou rallongé.

5. Dispositif de distraction selon la revendication 4, **caractérisé en ce que** dans la zone de l'élément de distraction respectif (5) il est réalisé un emplacement de séparation (38) dans la zone de laquelle la section d'extrémité (4) est amovible de la section médiane (3).

6. Dispositif de distraction selon la revendication 1, **caractérisé en ce que** la seconde partie de dispositif (2) est réalisée comme une charnière (14) avec deux moitiés de charnière (10, 11) une moitié de charnière (10) étant associée à l'os de menton et l'autre moitié de charnière (11) étant associé au segment d'os frontal à distracter.

7. Dispositif de distraction selon la revendication 6, **caractérisé en ce que** l'axe de charnière (21) s'étend parallèlement au plan d'occlusion et perpendiculaire au plan sagittal.

8. Dispositif de distraction selon la revendication 6, **caractérisé en ce que** la charnière (14) présente une butée (15) pour délimiter la zone de pivotement des moities de charnière (10, 11).

9. Dispositif de distraction selon la revendication 6, **caractérisé en ce que** les parties de fixations sont forme's par des perçages.

10. Dispositif de distraction selon la revendication 9, **caractérisé en ce que** les perçages sont formés par un trou oblong (12).

11. Dispositif de distraction selon la revendication 10, **caractérisé en ce que** le trou oblong (12) est réalisé par des perçages (16) se chevauchant partiellement de sorte que des positions de fixation discrètes sont formées.

12. Dispositif de distraction selon la revendication 10, **caractérisé en ce que** il est inséré dans le trou oblong (12) une gaine de guidage (17) qui est coulissante le long du trou oblong (12) dans la gaine de guidage (17) une vis de fixation étant insérable.

13. Dispositif de distraction selon la revendication 12, **caractérisé en ce que** la gaine de guidage (17) peut être coincée (19, 20) dans le trou oblong (12).

14. Dispositif de distraction selon la revendication 12, **caractérisé en ce que** sur la gaine de guidage (17) viens s'appliquer un câble de traction au moyen duquel la branche (11) de la seconde partie de dispositif (2), portant cette gaine de guidage (17), est pivotante autour de l'axe de charnière (21).

15. Dispositif de distraction selon la revendication 14, **caractérisé en ce que** le câble de traction peut être serré avec un élément de vis (24) maintenu dans un palier.

16. Dispositif de distraction selon la revendication 6, **caractérisé en ce que** la moitié de charnière (10) est en forme de V, les pièces de fixation étant prévues respectivement aux extrémités libres des deux branches.

17. Dispositif de distraction selon la revendication 14, **caractérisé en ce qu'**un point de fixation supplémentaire est prévu au point de liaison des deux branches.
